# EUROPEAN PATENT APPLICATION

(11) **EP 1 757 285 A1**
(43) Date of publication of application: **28.02.2007**
(21) Application number: 05734654.6
(22) Date of filing: 21.04.2005
(51) Int. Cl.: A61K 31/335, A61P 11/02, A61P 37/08, C07D 313/12

(54) **PREVENTIVE AND/OR THERAPEUTIC AGENT FOR CHRONIC SINUSITIS**

(30) Priority: 21.04.2004 JP 2004125487; 20.12.2004 JP 2004367288
(71) Applicant: KYOWA HAKKO KOGYO CO., LTD., Chiyoda-ku, Tokyo 100-8185 (JP)
(72) Inventor: YASUMOTO, Kazuhiko, Fukuoka 815-0074 (JP); SUOYA, Yuji, Nagasaki 857-0028 (JP); KAWAMOTO, Yuji c/o Osaka Branch Office, Kita-ku, Osaka shi, Osaka 530-8280 (JP); SUGIMACHI, Naho, Fukuoka-shi, Fukuoka 813-0003 (JP)
(74) Representative: Casalonga, Axel
(86) International application number: PCT/JP2005/007625
(87) International publication number: WO 2005/102313

(57) **Abstract**

The present invention provides a preventive and/or therapeutic agent and the like for chronic sinusitis, which comprises, as an active ingredient, a dibenzo[b,e]oxepine derivative represented by formula (I): 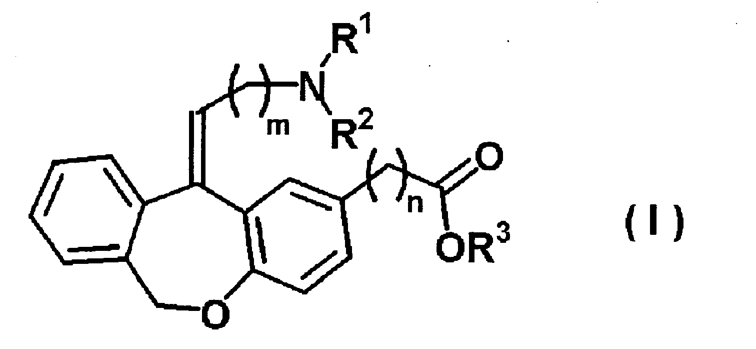 (wherein R¹, R², and R³ may be the same or different and each represents a hydrogen atom or lower alkyl, and m and n may be the same or different and each represents an integer of 1 to 4) or a pharmaceutically acceptable salt thereof.

## Description

### Technical Field

The present invention relates to a preventive and/or therapeutic agent for chronic sinusitis.

### Background Art

The paranasal sinuses are cavities in the bones around the nasal cavity consisting of the maxillary, ethmoidal, frontal, and sphenoidal sinuses which communicate with the nasal cavity. Sinusitis is nonallergic inflammation due to viral, bacterial, mycotic infection or the like, or allergic inflammation due to an allergic reaction in at least one of these sinuses. In particular, chronic or recurrent sinusitis is called "chronic sinusitis" (refer to Merck Index, 17th edition, section 7, chapter 86, etc.).

Chronic inflammation thickens the paranasal sinus mucosa (mucosal thickening) and narrows the external openings to the nasal cavity, thereby accumulating the secreted mucus in the paranasal sinuses. In addition, the thickened mucosa partially becomes a mass and forms a nasal polyp (polyp) to clog the nasal cavity. The symptoms of chronic sinusitis include nasal drip (prenasal drip), nasal discharge, nasal obstruction (nasal congestion), closure of the olfactory cleft, and the like. Furthermore, patients with chronic sinusitis sometimes complain of symptoms other than the nasal symptoms, such as a headache, a facial pain, fatigue, drop in concentration power, a sleep disorder, a taste disorder, a smell disorder, and the like. Furthermore, in patients with chronic sinusitis, inflammation in the nasal cavity and paranasal sinuses may spread to the middle ear to cause otitis media, or the nasal discharge (postnasal drip) flowing down into the fauces to cause chronic laryngopharyngitis, chronic bronchitis, or the like.

Examples of allergic chronic sinusitis include allergic chronic sinusitis accompanying aspirin asthma, allergic chronic sinusitis accompanying allergic rhinitis, and the like.

In addition, pathologic conditions of chronic sinusitis have been recently diversified, and eosinophilic infiltration of the mucosa has remarkably occurred regardless of the presence of an allergic reaction. Furthermore, presence of intractable sinusitis having the resistance to a macrolide therapy has been recognized. Examples of such sinusitis include chronic sinusitis accompanying eosinophilic sinusitis, chronic sinusitis accompanying bronchial asthma, and the like (Otorhinolaryngology, Tokyo, 2001, vol. 44, pp. 195-201 and Otorhinolaryngology, Tokyo, 2002, Vol. 45, pp. 239-241).

Chronic sinusitis is treated by a conservative treatment such as medication, lavage of the nasal sinuses, aspiration of nasal discharge, or a nebulizer treatment. However, it is difficult to completely treat chronic sinusitis by several times of ambulatory treatments, and the treatment period is generally increased. Furthermore, the symptoms do not improve by 2 to 6 months of such a conservative treatment, and a surgical operation may be required for a high degree of a symptom or mucosal inflammation. Known examples of a medication include a treatment using a small amount of 14-membered ring macrolide antibiotic (such as erythromycin, clarithromycin, roxithromycin) and the like for a long period of time and the like (Journal of Nippon Medical School, 1999, vol. 66, p.406).

On the other hand, dibenzo[b,e]oxepine derivatives or pharmaceutically acceptable salts thereof are known as compounds having, for example, an antiallergic function, an antiinflammatory function, an antiasthmatic function, and the like (refer to Patent Documents 1, 2, 3, 4, 5, and 6, and Non-patent Document 1).

In particular, (Z)-11-(3-dimethylaminopropylidene)-6,11-dihydrodibenzo[b,e]oxepine-2-acetic acid (hereinafter referred to as "compound 1") is known to have a histamine antagonism (refer to Non-patent Documents 2 and 3), an inhibitory action on chemical transmitter liberation (refer to Non-patent Document 4), an inhibitory action on inflammatory cytokine liberation (refer to Non-patent Document 5), an inhibitory action on tachykinin liberation (refer to Non-patent Document 6), and the like, and is clinically used as an antiallergic agent. It has also been found from a Phase III test that compound 1 has the function to highly improve the nasal congestion of allergic rhinitis (refer to Non-patent Document 7). It has been further known from a basic experiment using guinea pigs that compound 1 has an inhibitory action on a nasal congestion reaction in an experimental allergic rhinitis model (refer to Non-patent Document 8).
[Patent Document 1] Japanese Published Examined Patent Application No. 86925/1993
[Patent Document 2] US Patent No. 1282365
[Patent Document 3] Japanese Published Unexamined Patent Application No. 150082/1981
[Patent Document 4] Japanese Published Unexamined Patent Application No. 126883/1983
[Patent Document 5] Japanese Published Unexamined Patent Application No. 227879/1984
[Patent Document 6] Japanese Published Unexamined Patent Application No. 28972/1985
[Non-patent Document 1] Journal of Medicinal Chemistry (J. Med. Chem.), 1978, vol. 21, pp. 633-639
[Non-patent Document 2] Clinical Pharmacology and Therapy (Yakuri to Rinsho), 1995, vol. 5, pp. 1817-1824
[Non-patent Document 3] Clinical Pharmacology and Therapy (Yakuri to Rinsho), 1995, vol. 5, pp. 1825-1835
[Non-patent Document 4] International Archives of Allergy and Immunology (Int. Arch. Allergy Immunol.), 1996, vol. 110, pp. 57-63
[Non-patent Document 5] Acta Ophthalmologica Scandinavia (Acta. Ophthalmol. Scand.), 1999, vol. 228, pp. 33-37
[Non-patent Document 6] Japanese Journal of Pharmacology (Jpn. J. Pharmacol.), 2001, vol. 117, pp. 967-973
[Non-patent Document 7] Otorhinolaryngology (Jibi), 1996, vol. 42, pp. 633-658
[Non-patent Document 8] Allergy in Practice (Arerugi no Rinsho), 2003, vol. 23, pp. 63-75

### [Disclosure of Invention]

### [Problem to be Solved by the Invention]

An object of the present invention is to provide a preventive agent and/or therapeutic agent for chronic sinusitis, which comprises, as an active ingredient, a dibenzo[b,e]oxepine derivative or a pharmaceutically acceptable salt thereof.

### [Means for Solving the Problem]

The present invention relates to the following items (1) to (21):
(1) A preventive and/or therapeutic agent for chronic sinusitis, which comprises, as an active ingredient, a dibenzo[b,e]oxepine derivative represented by formula (I) (hereinafter referred to as "compound (I)): 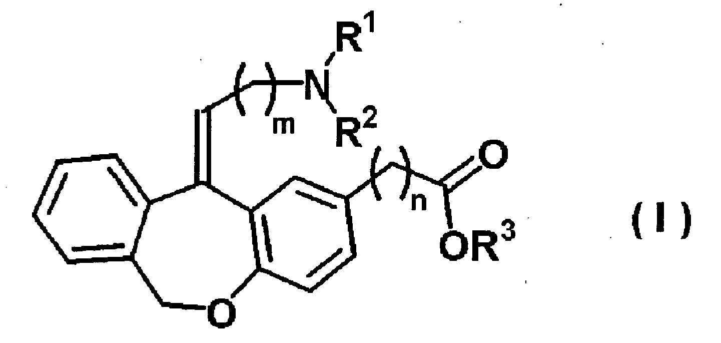 (wherein R¹, R², and R³ may be the same or different and each represents a hydrogen atom or lower alkyl, and m and n may be the same or different and each represents an integer of 1 to 4) or a pharmaceutically acceptable salt thereof.
(2) A preventive and/or therapeutic agent for chronic sinusitis, which comprises, as an active ingredient, (Z)-11-(3-dimethylaminopropylidene)-6,11-dihydrodibenzo[b,e]oxepine-2-acetic acid represented by formula (IA): 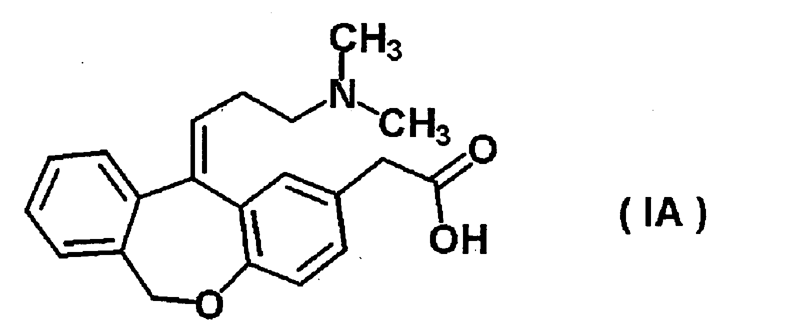 or a pharmaceutically acceptable salt thereof.
(3) The preventive and/or therapeutic agent for chronic sinusitis according to (1) or (2), wherein the chronic sinusitis is allergic chronic sinusitis.
(4) The preventive and/or therapeutic agent for chronic sinusitis according to (1) or (2), wherein the chronic sinusitis is nonallergic chronic sinusitis.
(5) An agent for improving a symptom of chronic sinusitis, which comprises, as an active ingredient, a dibenzo[b,e]oxepine derivative represented by formula (I): 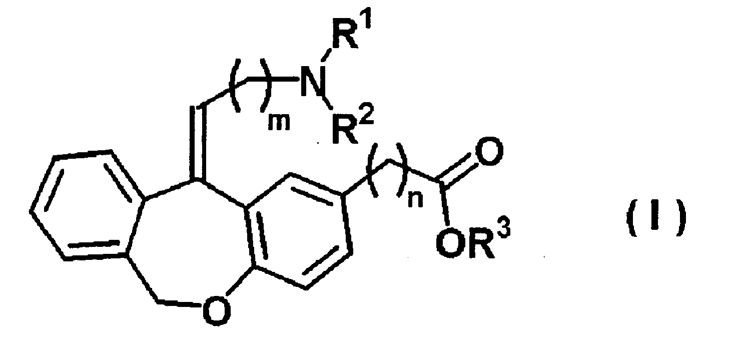 (wherein R¹, R², R³, m, and n have the same definitions as described above, respectively) or a pharmaceutically acceptable salt thereof.
(6) An agent for improving a symptom of chronic sinusitis, which comprises, as an active ingredient, (Z)-11-(3-dimethylaminopropylidene)-6,11-dihydrodibenzo[b,e]oxepine-2-acetic acid represented by formula (IA): 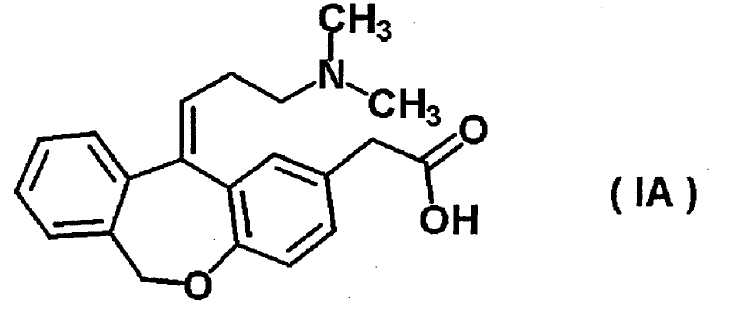 or a pharmaceutically acceptable salt thereof.
(7) The agent for improving a symptom of chronic sinusitis according to (5) or (6), wherein the chronic sinusitis is allergic chronic sinusitis.
(8) The agent for improving a symptom of chronic sinusitis according to (5) or (6), wherein the chronic sinusitis is nonallergic chronic sinusitis.
(9) The agent for improving a symptom of chronic sinusitis according to any one of (5) to (8), wherein the symptom is swelling of the inside wall of the nasal cavity.
(10) The agent for improving a symptom of chronic sinusitis according to any one of (5) to (8), wherein the symptom is nasal drip.
(11) The agent for improving a symptom of chronic sinusitis according to any one of (5) to (8), wherein the symptom is postnasal drip.
(12) The agent for improving a symptom of chronic sinusitis according to any one of (5) to (8), wherein the symptom is a headache.
(13) The agent for improving a symptom of chronic sinusitis according to any one of (5) to (8), wherein the symptom is a facial pain.
(14) The agent for improving a symptom of chronic sinusitis according to any one of (5) to (8), wherein the symptom is nasal mucosal expansion.
(15) The agent for improving a symptom of chronic sinusitis according to any one of (5) to (8), wherein the symptom is nasal obstruction.
(16) The agent for improving a symptom of chronic sinusitis according to any one of (5) to (8), wherein the symptom is inflammation recognized as a shadow in a plain radiograph.
(17) The agent for improving a symptom of chronic sinusitis according to any one of (5) to (8), wherein the symptom is nasal mucosal thickening or a nasal polyp.
(18) A preventive and/or therapeutic method for chronic sinusitis, which comprises administering an effective amount of a dibenzo[b,e]oxepine derivative represented by formula (I) : 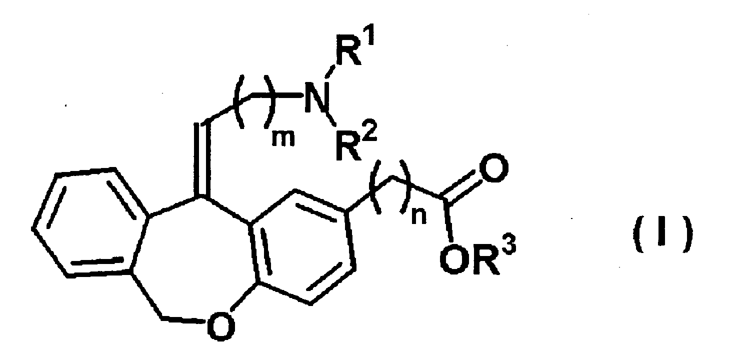 (wherein R¹, R², and R³ may be the same or different and each represents a hydrogen atom or lower alkyl, and m and n may be the same or different and each represents an integer of 1 to 4) or a pharmaceutically acceptable salt thereof.
(19) A preventive and/or therapeutic method for chronic sinusitis, which comprises administering an effective amount of (Z)-11-(3-dimethylaminopropylidene)-6,11-dihydrodibenzo[b,e]oxepine-2-acetic acid represented by formula (IA): 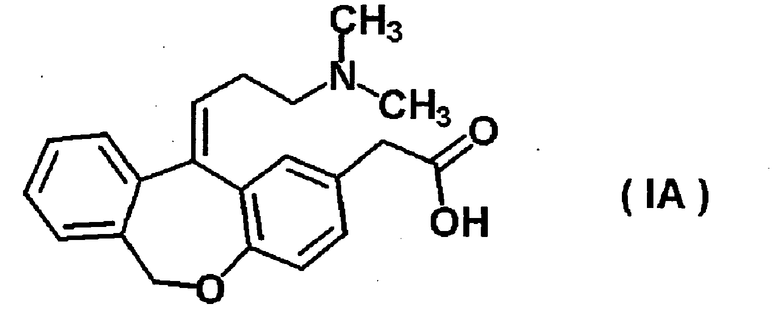 or a pharmaceutically acceptable salt thereof.
(20) Use of a dibenzo[b,e]oxepine derivative represented by formula (I): 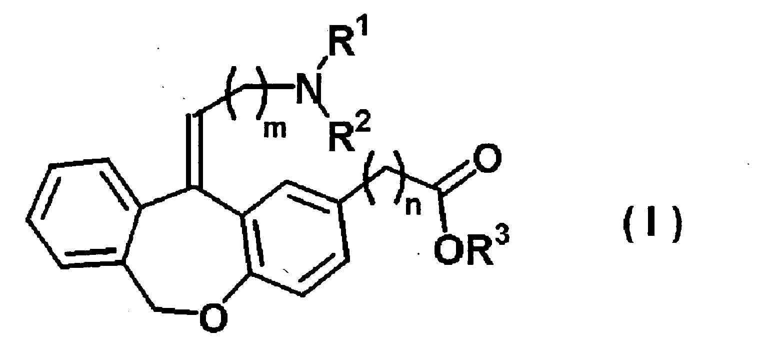 (wherein R¹, R², and R³ may be the same or different and each represents a hydrogen atom or lower alkyl, and m and n may be the same or different and each represents an integer of 1 to 4) or a pharmaceutically acceptable salt thereof, for the manufacture of a preventive and/or therapeutic agent for chronic sinusitis.
(21) Use of (Z)-11-(3-dimethylaminopropylidene)-6,11-dihydrodibenzo[b,e]oxepine-2-acetic acid represented by formula (IA): 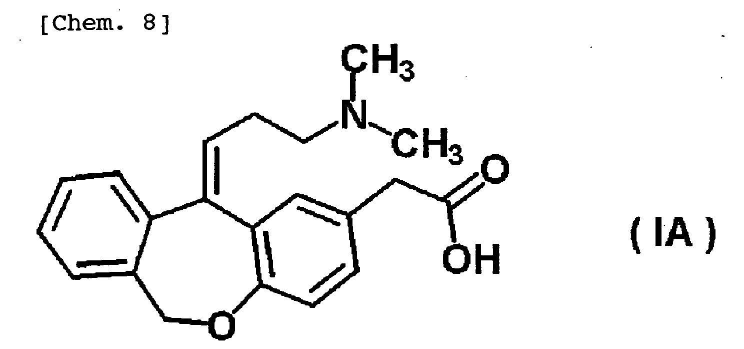 or a pharmaceutically acceptable salt thereof, for the manufacture of a preventive and/or therapeutic agent for chronic sinusitis.

### [Effect of Invention]

The present invention provides a preventive agent and/or therapeutic agent and the like for chronic sinusitis, which comprises, as an active ingredient, a dibenzo[b,e]oxepine derivative or a pharmaceutically acceptable salt thereof.

### [Best Mode for Carrying Out the Invention]

Examples of lower alkyl in the definition of each group in formula (I) include linear or branched alkyl having 1 to 8 carton atoms, such as methyl, ethyl, propyl, isopropyl, butyl, isobutyl, sec-butyl, tert-butyl, pentyl, hexyl, heptyl, octyl, and the like.

Examples of pharmaceutically acceptable salts of compound (I) or compound 1 include pharmaceutically acceptable acid addition salts, metal salts, ammonium salts, organic amine addition salts, amino acid addition salts, and the like. Examples of pharmaceutically acceptable acid addition salts of compound (I) or compound 1 include inorganic acid salts, such as hydrochlorides, sulfates, and phosphates; organic acid salts, such as acetates, maleates, fumarates, tartrates, citrates, and methanesulfonates.

Examples of pharmaceutically acceptable metal salts include alkali metal salts, such as sodium salts, and potassium salts; alkaline-earth metal salts, such as magnesium salts, and calcium salts; aluminum salts; zinc salts and the like. Examples of pharmaceutically acceptable ammonium salts include ammonium salts, tetramethylammonium salts, and the like. Examples of pharmaceutically acceptable organic amine addition salts include addition salts of morpholine, piperidine, and the like. Examples of pharmaceutically acceptable amino acid addition salts include addition salts of lysine, glycine, phenylalanine, aspartic acid, glutamic acid, and the like. Among these salts, monohydrochlorides are more preferred.

Although a method for producing compound (I) or compound 1 is not particularly limited, compound (I) or compound 1 can be obtained by the method described in, for example, Japanese Published Examined Patent Application No. 86925/1993 or a similar method. The resulting intermediate can be supplied to a subsequent reaction without particular purification.

The aimed compound of the production method can be isolated and purified by subjecting to a purification method generally used in organic synthetic chemistry, such as filtration, extraction, washing, drying, concentration, recrystallization, and various types of chromatography.

To obtain a salt of compound (I) or compound 1, when compound (I) or compound 1 is obtained in the form of a salt, the salt may be purified as it is. Further, when compound (I) or compound 1 is obtained in a free form, compound (I) or compound 1 may be dissolved or suspended in a suitable solvent, followed by addition of an acid or a base to form a salt.

Compound (I) or compound 1 and pharmaceutically acceptable salts thereof may exist in the form of an adduct with water or any of various solvents. The adduct can also be used for the preventive and/or therapeutic agent for chronic sinusitis of the present invention.

Examples of chronic sinusitis prevented and/or treated in the present invention include nonallergic chronic sinusitis due to viral, bacterial, mycotic inflection, or the like, allergic chronic sinusitis accompanying aspirin asthma, allergic chronic sinusitis accompanying allergic rhinitis such as chronic sinusitis complicated with perennial allergic rhinitis, chronic sinusitis accompanying eosinophilic sinusitis, intractable sinusitis and the like such as chronic sinusitis accompanying bronchial asthma, and the like. The preventive and/or therapeutic agent for chronic sinusitis of the present invention can prevent, improve, or treat various symptoms of chronic sinusitis (for example, nasal symptoms such as nasal mucosal thickening, polyp, nasal drip, nasal discharge, nasal obstruction, closure of olfactory cleft, and postnasal drip; symptoms such as a headache, a facial pain, fatigue, drop in concentration power, a sleep disorder, a taste disorder, and a smell disorder; and secondary symptoms such as otitis media, chronic laryngopharyngitis, and chronic bronchitis).

Compound (I), compound 1, or a pharmaceutically acceptable salt thereof can be administered alone but is preferably provided as various general pharmaceutical preparations. The pharmaceutical preparations are used for animals and humans.

A pharmaceutical preparation according to the present invention may comprise, as an active ingredient, compound 1 or a pharmaceutically acceptable salt thereof alone or as a mixture with any other effective ingredients. In addition, such a pharmaceutical preparation is prepared by mixing the active ingredient with one or more pharmaceutically acceptable carriers and then subjecting the resulting mixture to any method well known in the technical field of pharmaceutics.

Examples of another active ingredient to be mixed include erythromycin, clarithromycin, roxithromycin, and the like.

As for the administration route, it is preferred to select the most effective route of administration. Examples of the administration route include oral administration and parenteral administration such as hypodermic administration, intravenous administration, intranasal administration, and the like.

Examples of a dosage form include a tablet, a powder, granules, syrup, an injection, and the like.

A liquid preparation suitable for oral administration, for example, syrup, can be produced using water, a saccharide such as sucrose, sorbit, fructose, glycol such as polyethylene glycol or propylene glycol, oil such as sesame oil, olive oil, or soybean oil, a preservative such as p-hydroxybenzoate, and a flavor such as a strawberry flavor or peppermint and the like. A tablet, a powder, granules and the like can be produced by using an excipient such as lactose, glucose, sucrose, or mannital, a disintegrator such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, and the like.

An injection for hypodermic or intravenous administration (including continuous administration) is prepared as, for example, a solution, a suspension, an emulsion, or a solid injection used by dissolution or suspension in a solvent when used. A solution, a suspension, or an emulsion is prepared by dissolving, suspending, or emulsifying compound (I), compound 1, or a pharmaceutically acceptable salt thereof in a solvent. Examples of the solvent include distilled water for injection, physiological saline, a buffer (such as phosphate buffer), vegetable oil, propylene glycol, polyethylene glycol, alcohols such as ethanol, and mixtures thereof. Such an injection may further contain a stabilizer, a solubilizer (such as glutamic acid, aspartic acid, Polysorbate 80 (trade mark), or the like), a suspending agent, an emulsifier, a soothing agent, a buffer, a preservative, or the like. In a final step, they are sterilized or subjected to aseptic operation to produce the injection. A sterile solid preparation, for example, a freeze-dried product, and the like may be produced so that it can be used by dissolution or suspension in sterile distilled water for injection or other solvent before administration.

An external preparation for intranasal administration is, for example, a liquid for external use and the like. Such an external preparation is prepared as a solution, a suspension, an emulsion, or a solid preparation used by dissolution or suspension in a solvent when used or the like. A solution, a suspension, or an emulsion can be prepared by dissolving, suspending, or emulsifying compound 1 or a pharmaceutically acceptable salt thereof in a solvent. Examples of the solvent include physiological saline, a buffer (such as phosphate buffer), propylene glycol, polyethylene glycol, and the like mixtures thereof. Such a liquid for external use may further contain a stabilizer, a solubilizer (such as glutamic acid or aspartic acid), a suspending agent (such as a polymer imparting viscosity, including Polysorbate 80 (trade mark), carboxymethyl cellulose, polyvinyl alcohol or the like), an emulsifier, a buffer, a preservative, or the like.

The doses and frequency of administration of compound (I), compound 1, or a pharmaceutically acceptable salt thereof may vary depending on the dosage form, the age and body weight of a patient, the nature or seriousness of a symptom to be treated, and the like. In oral administration, in general, a dose of 0.01 mg to 1 g, preferably 0.05 to 50 mg, is administered to an adult once or several times a day. In parenteral administration such as intravenous administration, a dose of 0.001 mg to 100 mg, preferably 0.01 mg to 10 mg, is administered to an adult once or several times a day. However, these dosages and frequencies of administration vary with the various conditions described above.

Next, the effect of compound (I) on chronic sinusitis will be described in detail below with reference to test examples.

### Test Example 1: Effect 1 of monohydrochloride (olopatadine hydrochloride) of compound 1 on chronic sinusitis

A patient (female, 51 years old) diagnosed as chronic sinusitis was orally daily administered with olopatadine hydrochloride (Allelock (trade mark) 5 mg tablet; Kyowa Hakko Kogyo Co., Ltd.) alone in a dose of one tablet two times (after breakfast and before bedtime) a day (10 mg/day) everyday. Before the administration of olopatadine hydrochloride and 2 months after the administration, computed tomography (CT) was performed to observe changes in symptoms. Before the administration of olopatadine hydrochloride, the right nasal cavity of the patient was filled with a polyp, and the symptoms such as a headache, right nasal obstruction, purulent nasal discharge, closure of the right olfactory cleft due to mucosal thickening were and the like were observed in the patient. In administration of Klaricid (trade mark) (general name: clarithromycin) and Neuzym (trade mark) (general name: lysozyme chloride) for 2 months or more, improvement of these symptoms were not observed.

It was confirmed by the CT image that the polyp and soft-tissue density mass which filled the right nasal cavity substantially disappeared after administration of olopatadine hydrochloride for 2 months. Also, the soft-tissue density area which filled in the right ethmoidal sinus, the right sphenoidal sinus, and the right frontal sinus substantially disappeared, and good airlation was recognized. Furthermore, swelling of the inside wall toward the nasal cavity side, closure of the right olfactory cleft due to mucosal thickening and the like were relieved.

The subjective symptoms such as nasal drip and headache also disappeared after administration of olopatadine hydrochloride for 2 months.

The above results indicate that olopatadine hydrochloride improved the subjective symptoms (such as nasal drip, and headache) of patients with chronic sinusitis and the findings (such as polyp, nasal obstruction, swelling of the inside wall toward the nasal cavity side and closure of the right olfactory cleft due to mucosal thickening) recognized in the CT image. Namely, it was found that olopatadine hydrochloride has a clinical effect on chronic sinusitis.

### Test Example 2: Effect of olopatadine hydrochloride on patient with chronic sinusitis complicated with perennial allergic rhinitis

Patients (24 patients, average age 33.8 years old, duration 61.5±59.7 days) with chronic sinusitis complicated with perennial allergic rhinitis were orally administered with olopatadine hydrochloride (Allelock (trade mark) 5 mg tablet; Kyowa Hakko Kogyo Co., Ltd.) in a dose of one tablet two times (after breakfast and before bedtime) a day (10 mg/day) everyday. In this case, Mucodyne (trade mark) (general name: carbocysteine), Dasen (trade mark) (general name: serrapeptase), Polaramine (trade mark) (general name: d-chlorpheniramine maleate), Klaricid (trade mark) (general name: clarithromycin) or the like may be combined. The combined use with such a medicine was filled in a survey sheet. Such a medicine had been given for treatment before this test. At the eighth week of the administration, degrees of improvement in 1) sneezing, 2) nasal drip, 3) nasal obstruction, 4) interference with daily life, 5) mucosal swelling, 6) headache and head heaviness, and 7) postnasal drip were examined for each patient. The degree of each symptom determined with reference according to "Nasal Allergy Diagnosis Guideline for 2002 (Revised Fourth Edition)", and the degree of improvement in each symptom was evaluated for each patient having a corresponding symptom before the administration on the basis of the four stages, i.e., disappearance, significant improvement, improvement, and no change. In the case of more than improvement (disappearance, significant improvement, or improvement), the rate of improvement was evaluated as "effective" and calculated as a rate (%) of an effective number of patients relative to the number of patients (number of cases) having a corresponding symptom before administration. The results are shown in Table 1.

**[Table 1]**

| Degree of improvement in symptoms of chronic sinusitis complicated with perennial allergic rhinitis | | | | | | |
|---|---|---|---|---|---|---|
| | Disappearance | Significant improvement | Improvement | No change | Number of cases | Rate of improvement |
| Sneezing | 8 | 0 | 3 | 1 | 12 | 91.7% |
| Nasal drip | 4 | 2 | 5 | 4 | 15 | 73.3% |
| Nasal obstruction | 4 | 3 | 7 | 2 | 16 | 87.5% |
| Interference with daily life | 3 | 3 | 8 | 2 | 16 | 87.5% |
| Mucosal Swelling | 0 | 1 | 13 | 2 | 16 | 87.5% |
| Headache, head heaviness | 5 | 0 | 0 | 0 | 5 | 100% |
| Postnasal drip | 3 | 0 | 0 | 0 | 3 | 100% |

The results shown in Table 1 reveal that olopatadine hydrochloride improves the symptoms observed in patients with chronic sinusitis complicated with perennial allergic rhinitis, such as sneezing, nasal drip, nasal obstruction, interference with daily life, mucosal swelling, headache and head heaviness, and postnasal drip. In other words, it was found that olopatadine hydrochloride has a clinical effect on chronic sinusitis complicated with perennial allergic rhinitis.

The results of Test Examples 1 and 2 indicate that compound 1 or a pharmaceutically acceptable salt thereof is effective in improvement and/or treatment of the symptoms of allergic chronic sinusitis such as chronic sinusitis complicated with perennial allergic rhinitis, and chronic sinusitis such as nonallergic chronic sinusitis.

### Test Example 3: Effect 2 of olopatadine hydrochloride on chronic sinusitis

A patient (31 years old) diagnosed as chronic sinusitis was administered with olopatadine hydrochloride (Allelock (trade mark) 5 mg tablet; Kyowa Hakko Kogyo Co., Ltd.) according to the prescription schedule below (21 days in total).

### <Prescription schedule>

### 1. First 7 days (co-administration)

Three medicines were dosed together according to the following prescription. Also, frequently used SG granules were also prescribed.

Olopatadine hydrochloride 5 mg tablet: oral dose of one tablet two times a day for 7 days (10 mg/day)

Gatiflo (trade mark) (general name: gatifloxacin) 200 mg tablet: oral dose of one tablet two times a day for 4 days (400 mg/day)

Mucodyne (trade mark) (general name: carbocysteine) 500 mg tablet: oral dose of one tablet three times a day for 7 days (1500 mg/day)

### 2. Next 14 days (single administration of olopatadine hydrochloride)

Furthermore, olopatadine hydrochloride was given according to the following prescription:
Olopatadine hydrochloride 5 mg tablet: oral dose of one tablet two times a day for 14 days (10 mg/day)
Plain radiography was conducted before the administration of the above medicines and 21 days after the administration to observe changes in the symptoms.

Before the administration, the subjective symptoms such as purulent nasal discharge, nasal obstruction, cheek pains (facial pain), and headache were observed in the patient, and in the plain radiograph, shadows indicating sinusitis inflammation were recognized in the ethmoidal sinus and the right and left maxillary sinuses.

In the first 7 days of co-administration, the nasal symptoms such as purulent nasal discharge and nasal obstruction were improved, and the cheek pains (facial pain) and headache disappeared. Furthermore, in the next 14 days of single administration of olopatadine hydrochloride, the nasal symptoms such as purulent nasal discharge and nasal obstruction disappeared, and nasal mucosal swelling and the like were unobserved. Therefore, the nasal conditions were recognized as normal. It was also recognized that the shadows recognized in the plain radiograph, which indicates sinusitis inflammation in the ethmoidal sinus and the right and left maxillary sinuses, completely disappeared after the administration of olopatadine hydrochloride for a total of 21 days.

The above-described results indicate that olopatadine hydrochloride improves the subjective symptoms of patients with chronic sinusitis (purulent nasal discharge, nasal obstruction, cheek pains, and headache) and the findings (sinusitis inflammation) recognized in the plain radiograph. In other words, it was found that olopatadine hydrochloride has a clinical effect on nonallergic chronic sinusitis.

### Test Example 4: Effect 3 of olopatadine hydrochloride on chronic sinusitis

A patient (54 years old) diagnosed as chronic sinusitis was administered with olopatadine hydrochloride (Allelock (trade mark) 5 mg tablet; Kyowa Hakko Kogyo Co., Ltd.) according to the prescription schedule below (35 days in total).

### <Prescription schedule>

### 1. First 7 days (co-administration)

Three medicines were dosed together according to the following prescription.

Olopatadine hydrochloride 5 mg tablet: oral dose of one tablet two times a day for 7 days (10 mg/day)

Gatiflo (trade mark) (general name: gatifloxacin) 200 mg tablet: oral dose of one tablet two times a day for 4 days (400 mg/day)

Mucodyne (trade mark) (general name: carbocysteine) 500 mg tablet: oral dose of one tablet three times a day for 7 days (1500 mg/day)

### 2. Next 14 days (co-administration)

Two medicines were further dosed together according to the following prescription.

Olopatadine hydrochloride 5 mg tablet: oral dose of one tablet two times a day for 14 days (10 mg/day)

Mucodyne (trade mark) 500 mg table: oral dose of one tablet three times a day for 14 days (1500 mg/day)

### 3. Next 14 days (single administration)

Furthermore, olopatadine hydrochloride was given according to the following prescription:
Olopatadine hydrochloride 5 mg table: oral dose of one tablet two times a day for 14 days (10 mg/day)
Plain radiography was conducted before the administration of the above medicines and 35 days after the administration to observe changes in the symptoms.

Before the administration, the subjective symptoms such as purulent nasal discharge, postnasal drip, malaise, and a left cheek pain (facial pain) were observed in the patient, and in the plain radiograph, shadows indicating sinusitis inflammation were recognized in the ethmoidal sinus and the right and left maxillary sinuses.

In the first 7 days of co-administration, postnasal drip was not improved, but purulent nasal discharge was decreased and substantially disappeared. In addition, the left cheek pain (facial pain) and malaise disappeared. Furthermore, in the next 14 days of co-administration, the nasal symptoms such as purulent nasal discharge and postnasal drip disappeared, and nasal mucosal swelling and the like also disappeared. Therefore, the nasal conditions were recognized as normal. Furthermore, in the single administration of olopatadine hydrochloride, all subjective symptoms disappeared. It was also recognized that the shadows observed in the plain radiograph, which indicates sinusitis inflammation in the ethmoidal sinus and the right and left maxillary sinuses, completely disappeared after the administration of olopatadine hydrochloride for a total of 35 days.

The above-described results indicate that olopatadine hydrochloride improves the subjective symptoms of patients with chronic sinusitis (purulent nasal discharge, postnasal drip, malaise, and a left cheek pain) and the findings (sinusitis inflammation) recognized in the plain radiograph. In other words, it was found that olopatadine hydrochloride has a clinical effect on nonallergic chronic sinusitis.

### Test Example 5: Effect of olopatadine hydrochloride on patient with chronic sinusitis complicated with allergic rhinitis

A patient (18 years old) with chronic sinusitis complicated with allergic rhinitis was orally administered with olopatadine hydrochloride (Allelock (trade mark) 5mg tablet; Kyowa Hakko Kogyo Co., Ltd.) alone in a dose of one tablet two times a day for 2 months (10 mg/day).

The patient had developed serious sneezing, nasal obstruction, and postnasal drip about 5 and half years ago. The patient discontinued treatment when the subjective symptoms were relieved by intermittent medication. However, improvements in the symptoms were not observed before the start of treatment by administration of the above olopatadine hydrochloride. In addition, in a nasal discharge eosinophil test (an intermediate state between clusters of eosinophils and observable weak dispersion of eosinophils in nasal discharge) and a scratch test (house dust, candida, cedar, and orchard grass), the patient was positive before the start of administration, and the chronic sinusitis of the patient was recognized to be complicated with allergic rhinitis.

Before the administration of olopatadine hydrochloride and 2 weeks after and 1 month after the administration, transitions of the subjective symptoms of the patient, such as sneezing, nasal discharge, nasal obstruction, and postnasal drip, and findings such as swelling of the inferior turbinate mucosa, watery secretions, and the color of the inferior turbinate mucosa (objective symptoms) were examined. The degree of each symptom was decided on the basis of Tables 3 and 4 in reference to "Nasal Allergy Diagnosis Guideline for 2002 (Revised Fourth Edition)". The results are shown in Table 2.

**[Table 2]**

| Transitions of symptoms of patient with chronic sinusitis complicated with allergic rhinitis | | | |
|---|---|---|---|
| | Before administration | Two weeks after administration | One month after administration |
| Sneezing | + | - | - |
| Nasal discharge | + | - | - |
| Nasal obstruction | ++ | - | - |
| Postnasal drip | ++ | + | - |
| Swelling of inferior turbinate mucosa | ++ | + | - |
| Watery secretion | + | + | - |
| Color of inferior turbinate mucosa | ++ | - | - |

**[Table 3]**

| Evaluation of subjective symptoms | | | | | |
|---|---|---|---|---|---|
| | ++++ | +++ | ++ | + | - |
| Sneezing (average number of fits per day) | 21 or more | 11 to 20 | 6 to 10 | 1 to 5 | 0 |
| Nasal discharge (average number of nose blows per day) | 21 or more | 11 to 20 | 6 to 10 | 1 to 5 | 0 |
| Nasal obstruction | Complete obstruction all day | Very severe nasal obstruction and mouth breathing for many hours of a day | Severe nasal obstruction and intermittent mouth breathing a day | Nasal obstruction without mouth breathing | No |
| Postnasal drip | | Always | Frequently | Sometimes | No |

**[Table 4]**

| Evaluation of objective symptoms | | | | |
|---|---|---|---|---|
| | +++ | ++ | + | - |
| Swelling of inferior turbinate mucosa | The middle turbinate was not observed. | Intermediate between (+++) and (+) | An area up to the center of the middle turbinate was observed. | No |
| Color of inferior turbinate mucosa | (Pale) | (Red) | (Light red) | (Normal) |
| Watery secretion | Filling | Intermediate between (+++) and (+) | Adhesion | No |

As a result, the subjective symptoms such as sneezing, nasal discharge, nasal obstruction, and postnasal drip, and findings such as swelling of the inferior turbinate mucosa, watery secretions, and the color of the inferior turbinate mucosa (objective symptoms) were improved with the passage of time, and these symptoms completely disappeared 2 weeks or 1 month later.

In addition, computed tomography (CT) was conducted before the administration of olopatadine hydrochloride and 1 month after and 2 months after the administration, and plain radiography (Waters method) was conducted before the administration of olopatadine hydrochloride and 2 months after the administration to observe changes in the symptoms.

It was confirmed from a plain radiograph that the shadows observed in the maxillary sinus, which indicates inflammation of chronic sinusitis before the administration, disappeared after the single administration of olopatadine hydrochloride for 2 months.

It was also recognized by a CT image that mucosal swelling in the ethmoidal sinus and the maxillary sinus were significantly relieved by the single administration of olopatadine hydrochloride for 1 month and substantially disappeared after the single administration of olopatadine hydrochloride for 2 months.

The above-described results indicate that olopatadine hydrochloride improves the subjective symptoms of patients of chronic sinusitis complicated with allergic rhinitis (such as sneezing, nasal discharge, nasal obstruction, and postnasal drip), the objective symptoms (such as swelling of the inferior turbinate mucosa, watery secretions, and the color of the inferior turbinate mucosa), the findings recognized in the CT image and plain radiograph (such as inflammation of chronic sinusitis and mucosal swelling in the ethmoidal sinus and the maxillary sinus). In other words, it was found that olopatadine hydrochloride has a clinical effect on chronic sinusitis complicated with allergic rhinitis.

The results of Test Examples 1 to 5 indicate that chronic sinusitis can be improved and/or treated by administration of olopatadine hydrochloride regardless of having allergic property or nonallergenic property.

Although the present invention will be described in detail below with reference to examples, the present invention is not limited to these examples.

### [Example 1]

Tablet: A tablet having the following composition is prepared by a conventional method.

| Prescription | |
|---|---|
| Monohydrochloride of compound 1: | 5 mg |
| Lactose: | 61 mg |
| Potato starch: | 30 mg |
| Polyvinyl alcohol: | 3 mg |
| Magnesium stearate: | 1 mg |
| | 100 mg |

### [Example 2]

Powder: A powder having the following composition is prepared by a conventional method.

| Prescription | |
|---|---|
| Compound 1: | 10 mg |
| Lactose: | 190 mg |
| | 200 mg |

### [Example 3]

Injection: An injection having the following composition is prepared by a conventional method.

| Prescription | |
|---|---|
| Compound 1: | 2 mg |
| Purified soybean oil: | 200 mg |
| Purified egg-yolk lecithin: | 24 mg |
| Glycerin for injection: | 50 mg |
| Distilled water for injection: | 1.72 mL |
| | 2.00 mL |

### [Example 4]

Syrup: Syrup having the following composition is prepared by a conventional method.

| Prescription | |
|---|---|
| Compound 1: | 300 mg |
| Purified sucrose: | 40 g |
| Methyl paraoxybenzoate: | 40 mg |
| Ethyl paraoxybenzoate: | 10 mg |
| Strawberry flavor: | 1 mL |
| Distilled water: | proper amount |
| | 100.00 Ml |

## Claims

1. A preventive and/or therapeutic agent for chronic sinusitis, which comprises, as an active ingredient, a dibenzo[b,e]oxepine derivative represented by formula (I): 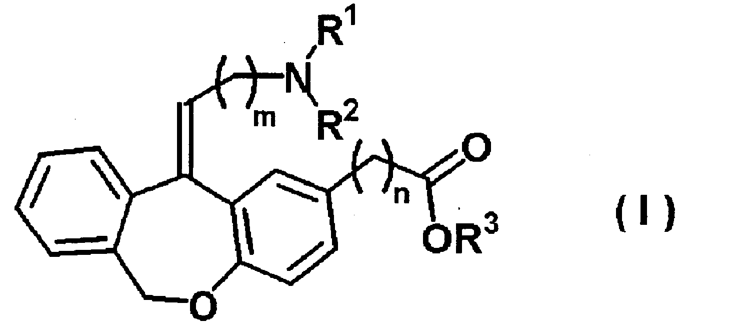 (wherein R¹, R², and R³ may be the same or different and each represents a hydrogen atom or lower alkyl, and m and n may be the same or different and each represents an integer of 1 to 4) or a pharmaceutically acceptable salt thereof.

2. A preventive and/or therapeutic agent for chronic sinusitis, which comprises, as an active ingredient, (Z)-11-(3-dimethylaminopropylidene)-6,11-dihydrodibenzo[b,e]oxepine-2-acetic acid represented by formula (IA): 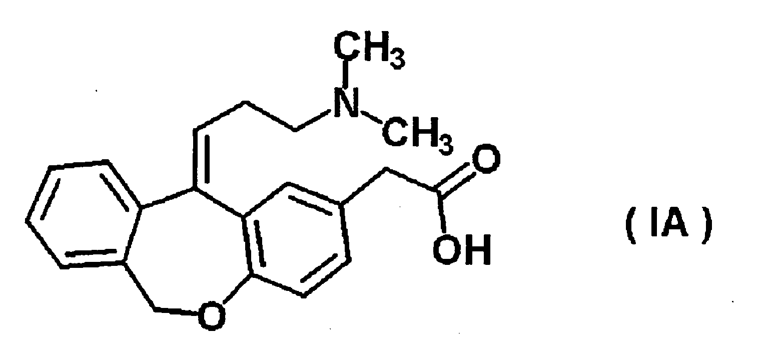 or a pharmaceutically acceptable salt thereof.

3. The preventive and/or therapeutic agent for chronic sinusitis according to Claim 1 or 2, wherein the chronic sinusitis is allergic chronic sinusitis.

4. The preventive and/or therapeutic agent for chronic sinusitis according to Claim 1 or 2, wherein the chronic sinusitis is nonallergic chronic sinusitis.

5. An agent for improving a symptom of chronic sinusitis, which comprises, as an active ingredient, a dibenzo[b,e]oxepine derivative represented by formula (I): 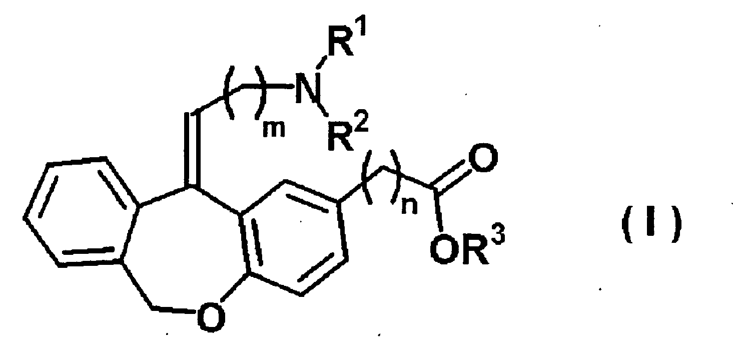 (wherein R¹, R², R³, m, and n have the same definitions as described above, respectively) or a pharmaceutically acceptable salt thereof.

6. An agent for improving a symptom of chronic sinusitis, which comprises, as an active ingredient, (Z)-11-(3-dimethylaminopropylidene)-6,11-dihydrodibenzo[b,e]oxepine-2-acetic acid represented by formula (IA): 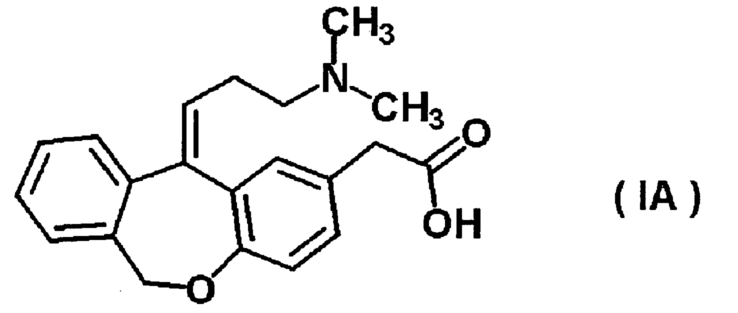 or a pharmaceutically acceptable salt thereof.

7. The agent for improving a symptom of chronic sinusitis according to Claim 5 or 6, wherein the chronic sinusitis is allergic chronic sinusitis.

8. The agent for improving a symptom of chronic sinusitis according to Claim 5 or 6, wherein the chronic sinusitis is nonallergic chronic sinusitis.

9. The agent for improving a symptom of chronic sinusitis according to any one of Claims 5 to 8, wherein the symptom is swelling of the inside wall of the nasal cavity.

10. The agent for improving a symptom of chronic sinusitis according to any one of Claims 5 to 8, wherein the symptom is nasal drip.

11. The agent for improving a symptom of chronic sinusitis according to any one of Claims 5 to 8, wherein the symptom is postnasal drip.

12. The agent for improving a symptom of chronic sinusitis according to any one of Claims 5 to 8, wherein the symptom is a headache.

13. The agent for improving a symptom of chronic sinusitis according to any one of Claims 5 to 8, wherein the symptom is a facial pain.

14. The agent for improving a symptom of chronic sinusitis according to any one of Claims 5 to 8, wherein the symptom is nasal mucosal expansion.

15. The agent for improving a symptom of chronic sinusitis according to any one of Claims 5 to 8, wherein the symptom is nasal obstruction.

16. The agent for improving a symptom of chronic sinusitis according to any one of Claims 5 to 8, wherein the symptom is inflammation recognized as a shadow in a plain radiograph.

17. The agent for improving a symptom of chronic sinusitis according to any one of Claims 5 to 8, wherein the symptom is nasal mucosal thickening or a nasal polyp.

18. A preventive and/or therapeutic method for chronic sinusitis, which comprises administering an effective amount of a dibenzo[b,e]oxepine derivative represented by formula (I): 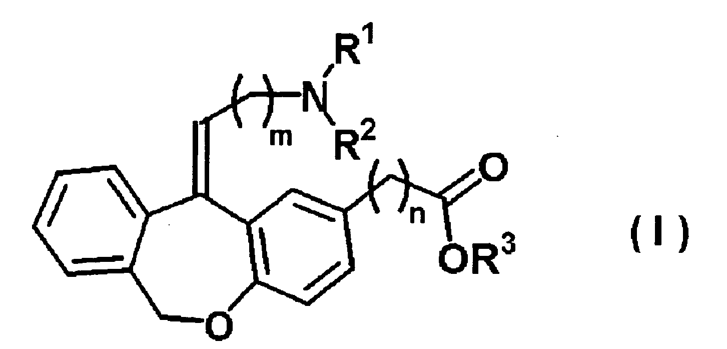 (wherein R¹, R², and R³ may be the same or different and each represents a hydrogen atom or lower alkyl, and m and n may be the same or different and each represents an integer of 1 to 4) or a pharmaceutically acceptable salt thereof.

19. A preventive and/or therapeutic method for chronic sinusitis, which comprises administering an effective amount of (Z)-11-(3-dimethylaminopropylidene)-6,11-dihydrodibenzo[b,e]oxepine-2-acetic acid represented by formula (IA): 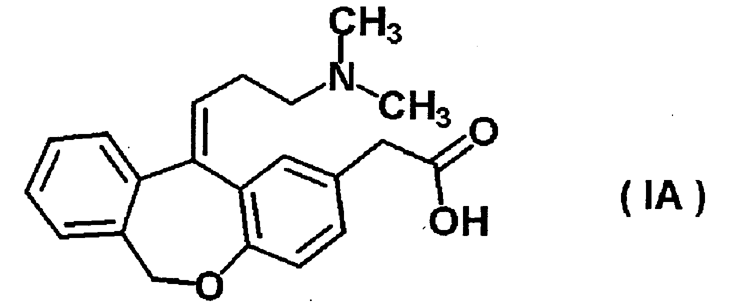 or a pharmaceutically acceptable salt thereof.

20. Use of a dibenzo[b,e]oxepine derivative represented by formula (I): 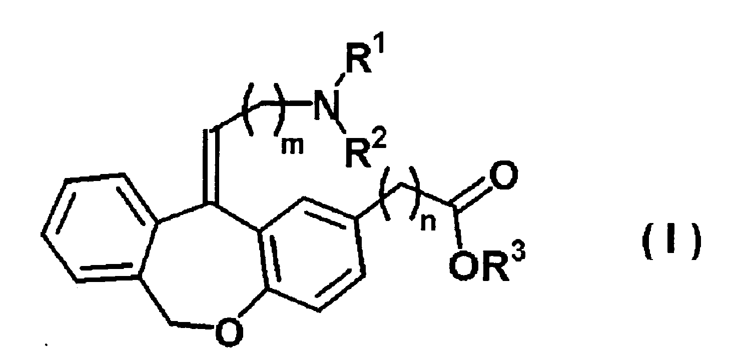 (wherein R¹, R², and R³ may be the same or different and each represents a hydrogen atom or lower alkyl, and m and n may be the same or different and each represents an integer of 1 to 4) or a pharmaceutically acceptable salt thereof, for the manufacture of a preventive and/or therapeutic agent for chronic sinusitis.

21. Use of (Z)-11-(3-dimethylaminopropylidene)-6,11-dihydrodibenzo[b,e]oxepine-2-acetic acid represented by formula (IA): 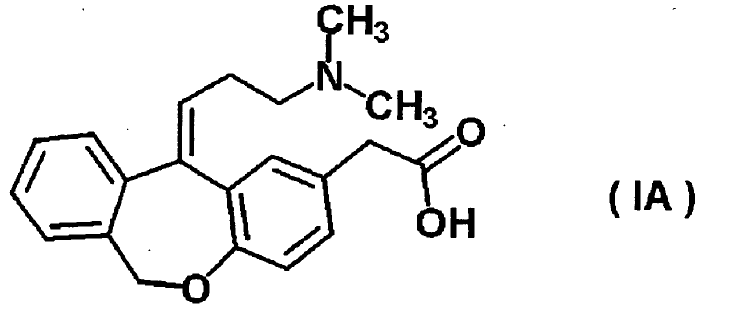 or a pharmaceutically acceptable salt thereof, for the manufacture of a preventive and/or therapeutic agent for chronic sinusitis.
